# EUROPEAN PATENT APPLICATION

(11) **EP 1 977 778 A1**
(43) Date of publication of application: **08.10.2008**
(21) Application number: 07300938.3
(22) Date of filing: 06.04.2007
(51) Int. Cl.: A61M 11/00, A61M 15/00

(54) **Electronic nebulizer for washing the nasal cavities of a user**

(71) Applicant: Markos Mefar S.P.A., 25073 Bovezzo (IT)
(72) Inventor: Giaretta, Mariano, Liscate (IT)
(74) Representative: Pittis, Olivier

(57) **Abstract**

A nebulizer for washing one or several nasal cavities of a user comprising a main body (20) having a proximal nasal portion (21) to be inserted into a nasal cavity of a user and comprising an outlet (22) situated at an extremity of said proximal nasal portion (21), a vessel (1) for receiving a liquid to be atomized, a pump device (23, 2, 3, 4, 5) comprising a pump shaft (23) having an upper end (2) and lower end (3), said lower end (3) being situated inside said vessel (1), and a pump bore (4) being axially arranged through said pump shaft (23) between the upper end (2) and the lower end (3), and further comprising a vibrator (5), a mesh member (6) having a rear surface (6b) and a front surface (6a), said rear surface (6b) facing the upper end (2) of the pump shaft (23), said mesh member (6) comprising at least one traversing hole (24) extending between said rear and front surfaces (6a, 6b), said pump shaft (23) being actuated by the vibrator (5) for vibrating said pump shaft (23) in such a way that the liquid pumped by the pump device (23, 2, 3, 4, 5) in the vessel (1) passes through said at least one hole (24) from the rear surface (6b) in the direction of the front surface (6a) of the mesh member (6), and is subsequently atomized in the proximal nasal portion (21) and delivered by the outlet (22) situated at an extremity of said proximal nasal portion (21), and a sealing element (7) being in contact with at least the front surface (6a) of the mesh member (6) and surrounding said mesh member (6), said sealing element (7) being arranged in a fixed position.

## Description

The present invention is directed to an electronic nebulizer for washing the nasal cavities of a human with saline solutions and for the administration of therapeutic agents, both topical and systemic, to the nasal cavity and paranasal sinuses of a human.

The goal of the washing with saline solutions of the nasal cavities of a person, such as a patient, is to dissolve the mucus and catarrh that are located in said nasal cavities, thereby helping that person to eliminate them and preparing the nasal cavities for the subsequence administration of therapeutic agents or drugs.

Said therapeutic agents can be subsequently administrated, both topical and systemic, with a greater efficiency to the nasal cavities and paranasal sinuses.

Indeed, without such pre-washing, the drugs or therapeutic agents do not act properly as they are blocked by the mucus and similar comprised in the nasal cavities, and hence can not reach the nasal areas where they are supposed to act and/or to enter into the human body, i.e. into the blood circulation.

For instance, million of people suffer from rhinopharyngitis, allergic rhinitis and chronic sinusitis and are treated by means of topically antihistamines, antibiotics and decongestants delivered intranasally.

In addition to saline solutions and topically applied drugs, there are a wide variety of systemically absorbed drugs that are delivered intranasally.

However, the known devices utilized so far to deliver saline solutions, topical drugs and systemic drugs have proven to have major disadvantages for the patient, such as local irritation, nosebleeds and uncomfortable taste from concentrated drugs reaching the mouth.

In addition, these prior art devices have proven to be extremely inadequate in reaching all areas needed, especially the deep nasal cavity and paranasal sinuses.

Current delivery systems comprise, for example, a pressurized canister that ejects the drug into the nostril in short bursts or streams of atomized liquid in an aqueous nasal spray, such as a metered dose inhaler (MDI).

However, even if these devices are compact, noiseless, easy portable and user friendly, the efficacy of drug administered in this manner is limited due to dose variability and inadequate distribution of the particles, which limit the clinical effects of topical drugs and bio-availability of systemic drugs.

Besides, jet nebulizers are devices for both pulmonary and nasal delivery which convert drugs into nebulized particles by means of the Venturi effect. Jet nebulizers for nasal delivery are characterized by a body comprising a Venturi system and an element shaped as a bell or "bell element" which delimits a nebulization chamber. In a specific embodiment, these devices further include a second "bell element" which, in combination with the first bell element, creates a chamber that serves to collect the liquid and residues.

However, in that case, the efficacy of the drug administered and the drug dose reproducibility are influenced by the pressure and flow rate that generates the Venturi effect, which could result in a inadequate distribution of the particles in the patient's airways. In addition, these devices are usually heavy and noisy.

Further, nebulizers using an electronic pump and a mesh member are also known. However, these devices are only useable for drug delivery to the lung of the patient, i.e. there are not adapted for nasal washing and for both systemical and topical drug delivery.

As a consequence, there is a need in the art for delivery devices that enable better delivery of drug as well as a delivery of a greater amount of drug to all areas of nasal cavity and/or paranasal sinuses.

Further, there is also a need for a device which is compact, noiseless, easy portable, patient friendly but, at the same time, able to delivery constant doses with an adequate distribution of the particles, minimizing the lower airways deposition.

A goal of the present invention is to provide an improved electronic nebulizer for washing the nasal cavities of a human with saline solutions and for the administration of therapeutic agents, both topical and systemic, to the nasal cavity and/or paranasal sinuses of a human, and that overcomes at least some, but preferably most of them or all, the drawbacks and problems of the devices of the prior art.

A solution according to the present invention is a nebulizer for washing one or several nasal cavities of a user comprising :
- a main body having a proximal nasal portion to be inserted into a nasal cavity of a user and comprising an outlet situated at an extremity of said proximal nasal portion,
- a vessel for receiving a liquid to be atomized,
- a pump device comprising a pump shaft having an upper end and lower end, said lower end being situated inside said vessel, and a pump bore being axially arranged through said pump shaft between the upper end and the lower end, and further comprising a vibrator,
- a mesh member having a rear surface and a front surface, said rear surface facing the upper end of the pump shaft, said mesh member comprising at least one traversing hole extending between said rear and front surfaces,
- said pump shaft being actuated by the vibrator for vibrating said pump shaft in such a way that the liquid pumped by the pump device in the vessel passes through said at least one hole from the rear surface in the direction of the front surface of the mesh member, and is subsequently atomized in the proximal nasal portion and delivered by the outlet situated at an extremity of said proximal nasal portion, and
- a sealing element being in contact with at least the front surface of the mesh member and surrounding said mesh member, said sealing element being arranged in a fixed position.

Further, the nebulizer of the present invention can comprise one or several of the additional following features :
- the sealing element is a gasket, preferably an O-ring.
- the gasket and the mesh member are maintained by connection means, preferably the connection means are formed in the internal wall of the proximal nasal portion of the main body.
- the connection means comprise a radial expansion of the internal wall of the proximal nasal portion of the main body and a recess arranged in said radial expansion for receiving the gasket and the mesh member.
- the radial expansion of the internal wall comprises at least one first air passage and the wall of the proximal nasal portion comprises at least a second air passage in fluid communication with the ambient atmosphere for equilibrating the gas pressure in the nasal cavity of the user with the atmospheric pressure when the proximal nasal portion is inserted in said nasal cavity.
- the rear surface of said mesh member is arranged in close proximity to the upper end of the pump shaft, preferably the mesh member is maintained in contact with the extremity of said upper end by means of the gasket.
- the lower end of the pump is in the deepest part of the liquid suction chamber of the vessel.
- at least one hole of the mesh member is configured in such a manner that the liquid passing through said hole produces a conical-shape nebulization, and the shape and size of said proximal nasal portion, and the position of the mesh member with respect to the outlet are chosen in order to completely eject the nebulization cone outside said proximal nasal portion through the outlet.
- the mesh member comprises several traversing holes extending between the rear and front surfaces of said mesh member.
- the upper part of said liquid suction chamber is shaped in order to be directly connected to a disposable cartridge for filling liquid into the suction chamber of a vessel.
- the proximal nasal portion is bell-shaped.
- the proximal nasal portion comprises an external and an internal coaxially-arranged bell-shaped portions spaced one from the other by an collecting chamber for collecting liquids and/or residues, preferably the internal bell-shaped portion comprises an opening for evacuating liquid and/or residues present in the internal bell-shaped portion of the proximal nasal portion into the collecting chamber.
- the main body further comprises an electronic unit for controlling the vibrator and a battery unit for supplying electric power at least to the vibrator.
- the mesh member is a plate.
- it comprises an ON/OFF mechanism adapted to activate the electronic unit.
- the pump device is an ultrasonic pump and/or the vibrator is an ultrasonic vibrator.
- the pump device comprises at least one piezo-electric element arranged on the shaft.
- the main body has a proximal nasal portion having a shape and size adapted for being, at least partially, inserted into a nasal cavity of the user.

An embodiment of a nebulizer according to the present invention has been represented on the illustrative Figures, among which :
- Fig. 1 represents a first embodiment of a nebulizer according to the present invention,
- Fig. 2a represents an enlarged view of the shaft of the nebulizer of Fig. 1,
- Fig. 2b represents an enlarged view of the proximal nasal portion of the nebulizer of Fig. 1,
- Fig. 3 represents an enlarged view of the liquid vessel of the nebulizer of Fig. 1,
- Fig. 4 represents an enlarged view of an alternative liquid vessel that can be arranged in the nebulizer of Fig. 1,
- Fig. 5 represents a second embodiment of the proximal nasal portion of a nebulizer according to the present invention,
- Fig. 6 represent several embodiments of a mesh member useable in a nebulizer according to the present invention, and
- Fig. 7a and 7b represent alternative embodiments of gaskets cooperating with the mesh member of a nebulizer according to the present invention.

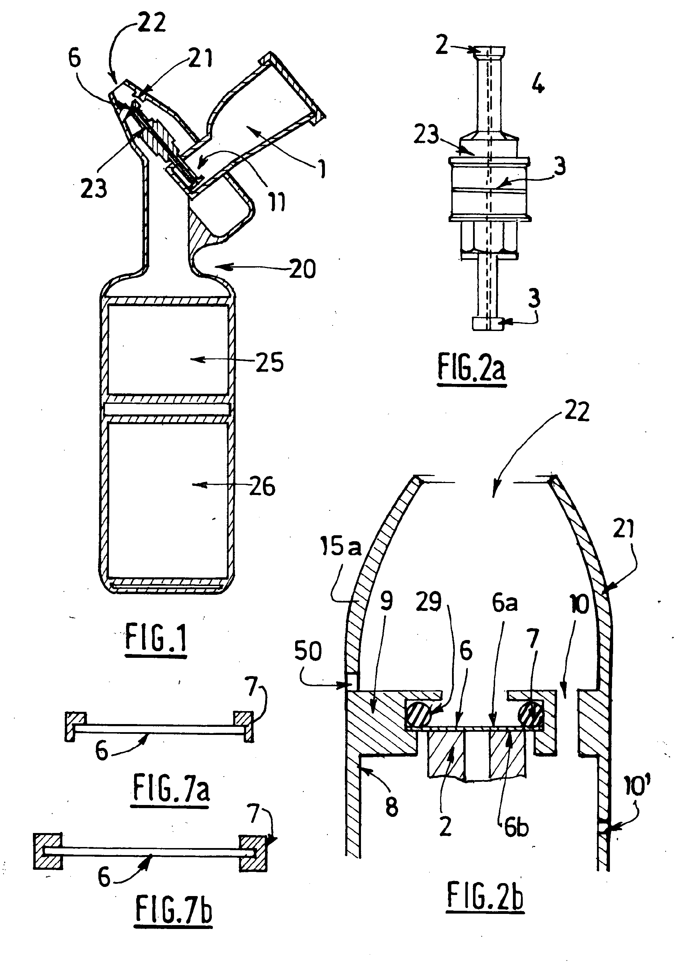

More precisely, Figure 1 represents a first embodiment of a nebulizer according to the invention useable for washing one or several nasal cavities or nostrils of a user using a liquid saline solution for instance.

It comprises a main body 20 having a proximal nasal portion 21 having, in this embodiment, a shape and size adapted for being, at least partially, inserted into a nasal cavity of a user. In other words, the shape and size of the proximal nasal portion 21 are chosen for allowing an efficient entering and positioning of said part in the nostrils of the user, i.e. the patient. Preferably, it is bell-shaped.

An outlet 22 situated at the extremity of the proximal nasal portion 21 that is inserted into the patient's nostrils, is used for delivering the atomized washing liquid in said nostrils, the liquid to be atomized being stored in a liquid storage vessel 1.

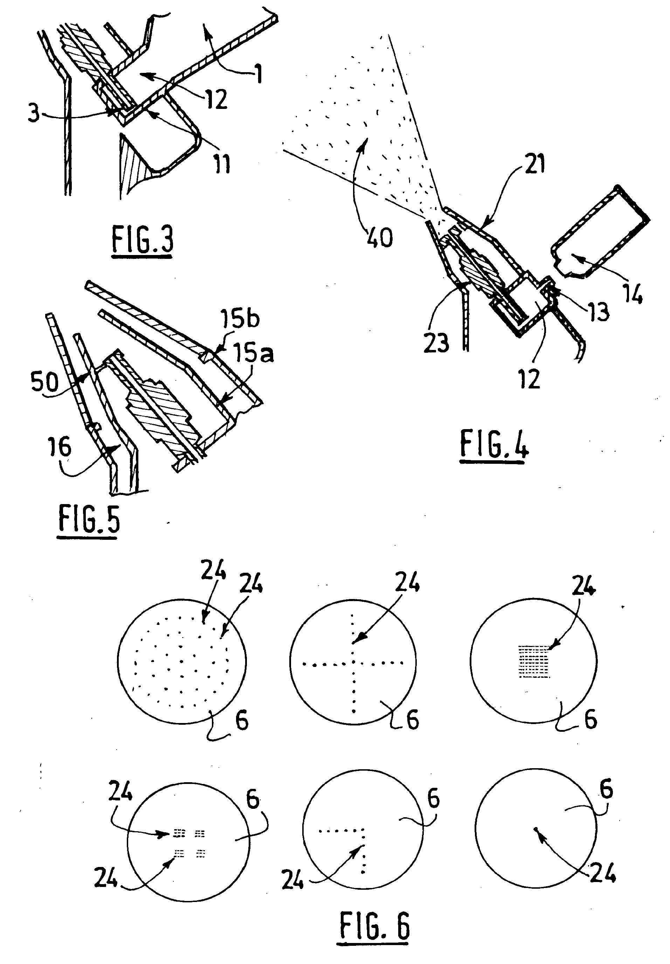

A pump device 23, 2, 3, 4, 5 is used for pumping the liquid into the storage container or vessel 1. Said pump device is preferably an ultrasonic pump as it allows to obtain a device which is noiseless, compact and able to better control the particle sizes and their distribution as a nebulization cone 40 as shown on Figure 4.

As illustrated on Figure 2a, said pump comprises a pump shaft 23 having an upper end 2 and lower end 3, and a pump bore 4 is axially arranged through the pump shaft 23 between both ends 2, 3. In other words, the pump shaft 23 is hollow as a tube.

A vibrator 5, preferably an ultrasonic vibrator, cooperates with the pump shaft 23 for vibrating said pump shaft 23, preferably upwardly and downwardly, in such a way that the liquid pumped by the pump device 23, 2, 3, 4 in the vessel 1 passes through said hole or holes 24 of the mesh member 6, is atomized in the proximal nasal portion 21 and subsequently delivered in the patient's nostril by the outlet 22 situated at the extremity of the proximal nasal portion 21.

The vibrator 5 comprises one or several piezo-electric elements, preferably annular-shaped piezo-electric elements arranged on the peripheral external surface of the shaft 23. Such arrangements are well-known in the art and do not need to be further explained in the frame of the present invention.

Preferably, the pump device 23, 2, 3, 4,5 is an ultrasonic pump and the vibrator 5 is an ultrasonic vibrator.

The lower end 3 of the shaft is situated inside said liquid storage vessel 1 in order to be able to withdraw by suction some washing liquid from the vessel 1 in order to minimize the amount of liquid that can not be pumped in the vessel 1. In other words, the lower end 3 of the pump 23 is in the deepest part 11 of the liquid suction chamber 12 of the vessel 1 in order to be able to withdraw by suction as much liquid as possible from the vessel 1 as shown on Figures 3 and 4.

Besides, as illustrated on Figure 2b, the upper end 2 of the shat 23 is arranged in close vicinity to a mesh member 6, preferably a plate or similar, having a rear surface 6b and a front surface 6a and comprising at least one, but preferably several, traversing hole or holes 24 extending through said mesh 6, between said rear and front surfaces 6a, 6b so that the washing liquid can pass trough said holes. Using a mesh member 6 and a vibrating shaft 23 for creating a nebulization jet is also well-known in the art and will not detailed here.

Other possible embodiments of such mesh plates 6 are shown on Figure 6. As one can see, the mesh plate 6 can comprise one or several traversing holes 24 that can be arranged in arrays, in circles, by groups etc... Further, the mesh member 6 is preferably a disk, but it could have another forms, such as square, rectangle, ellipse...

The position of the holes 24 in the mesh member 6 is a function of the areas of the nasal cavity intended to be reached, whereas the number of holes 24 in the mesh member 6 is function of the nebulization speed intended to be reached. Preferably, the hole or holes 24 of the mesh member 6 are configured in such a manner that the liquid passing through said holes produces a conical-shape 40 nebulization, and the shape and size of said proximal nasal portion 21, and the position of the mesh member 6 with respect to the outlet 22 are chosen in order to completely eject the nebulization cone outside said proximal nasal portion 21 through the outlet 22 and subsequently in the nostrils of the patient. These parameters can be chosen by the man of skilled in the art upon the medical specifications to be reached.

Besides, the mesh member 6 can be a metal plate, such as stainless steel, nickel-palladium..., or made of a polymere material.

An important feature of the present invention is that a sealing element, i.e. a gasket 7, such as an O-ring, is positioned on the front surface 6a of the mesh member 6, while being in contact with said front surface 6a of the mesh member 6 and surrounding said mesh member 6, i.e. in contact with the disk periphery of the mesh member. The gasket 7 is arranged and maintained in a fixed position, i.e. it is not mobile.

The role of said gasket 7 is to avoid liquid leaks between the periphery of the mesh member 6 and the internal wall 8 of the main body 20, i.e. to avoid the passage of liquid and/or residues under the mesh member 6. The gasket 7 is preferably an O-ring as shown on Figure 2, but can have other suitable shapes as represented in cross-section on Figures 7a and 7b.

The gasket 7, such as an O-ring, and the mesh member 6 are maintained by connection means 8, 9, that are preferably formed in the internal wall 8 of the proximal nasal portion 21. That is to say, a part such as a radial expansion 9 of the internal wall 8 of the main body comprises a recess 29 or a groove arranged in said radial expansion 9 or shoulder for receiving the gasket 7 and the mesh member 6.

The sealing element 7 arranged on the mesh member 6 is a gasket, preferably an O-ring as illustrated on Figure 2, but it can also have a different form, such as represented for instance on Figures 7a and 7b.

According to another embodiment shown on Figure 4, the upper part 13 of the liquid suction chamber 12 can be shaped for been directly connectable to a disposable liquid cartridge 14 for re-filling the liquid vessel 1.

The proximal nasal portion 21 is preferably bell-shaped to be easily inserted into the patient's nostril as shown on Figure 1.

However, in the embodiment shown on Figure 5, the proximal nasal portion 21 can be also formed of two coaxial bell-shaped portions 15a, 15b, i.e. an external 15b and an internal 15a bell-shaped portions, spaced one from the other by an collecting chamber 16 for collecting liquids and/or residues that are evacuated from the nasal cavities of the patient, i.e. the mixture of saline solution, mucus...., between the two coaxial bell-shaped portions 15a, 15b. Further, the internal bell-shaped portion 15a of the proximal nasal portions comprises preferably one (or several) opening 50 for also evacuating any liquid and/or residues passing into said internal bell-shaped portion 15a into the collecting chamber 16, during a nasal washing operation, which avoids any accumulation of liquid and residues in the area situted above the mesh member 6 in the proximal nasal portion. In this embodiment, it is the external bell-shaped portion 15b that is in contact with the patient's nasal cavity.

Besides, as shown on Figure 2b, the radial expansion or shoulder 9 of the internal wall 8 of the proximal nasal portion 21 comprises at least one first air passage 10 and the wall 8 of the proximal nasal portion 21 comprises at least a second air passage 10' in fluid communication with the ambient atmosphere which are used for equilibrating the gas pressure in the nasal cavity of the user with the atmospheric pressure when the proximal nasal portion 21 is inserted in said nasal cavity, i.e. during a nasal washing operation.

An electronic unit 25 for controlling the vibrator 5 and a battery unit 26 for supplying electric power at least to the vibrator 5 are also arranged in the nebulizer of the invention, as visible on Figure 1. It also comprises an ON/OFF mechanism (not shown of the Figures) for allowing or not the electric current to reach the vibrator 5 and/or the electronic unit 25. Here again, electronic unit 25, battery unit 26 and ON/OFF mechanism are well-known in the art and do not need to be further detailled.

The nebulizer of the present invention can be used for washing one or several nasal cavities of a user by injecting in said nasal cavity or cavities an atomized jet of a saline solution containing or not one or several therapeutic agents.

## Claims

1. Nebulizer for washing one or several nasal cavities of a user comprising :
- a main body (20) having a proximal nasal portion (21) to be inserted into a nasal cavity of a user and comprising an outlet (22) situated at an extremity of said proximal nasal portion (21),
- a vessel (1) for receiving a liquid to be atomized,
- a pump device (23, 2, 3, 4, 5) comprising a pump shaft (23) having an upper end (2) and lower end (3), said lower end (3) being situated inside said vessel (1), and a pump bore (4) being axially arranged through said pump shaft (23) between the upper end (2) and the lower end (3), and further comprising a vibrator (5),
- a mesh member (6) having a rear surface (6b) and a front surface (6a), said rear surface (6b) facing the upper end (2) of the pump shaft (23), said mesh member (6) comprising at least one traversing hole (24) extending between said rear and front surfaces (6a, 6b),
- said pump shaft (23) being actuated by the vibrator (5) for vibrating said pump shaft (23) in such a way that the liquid pumped by the pump device (23, 2, 3, 4, 5) in the vessel (1) passes through said at least one hole (24) from the rear surface (6b) in the direction of the front surface (6a) of the mesh member (6), and is subsequently atomized in the proximal nasal portion (21) and delivered by the outlet (22) situated at an extremity of said proximal nasal portion (21), and
- a sealing element (7) being in contact with at least the front surface (6a) of the mesh member (6) and surrounding said mesh member (6), said sealing element (7) being arranged in a fixed position.

2. Nebulizer according to claim 1, wherein the sealing element (7) is a gasket, preferably an O-ring.

3. Nebulizer according to claim 1 or 2, wherein the gasket (7) and the mesh member (6) are maintained by connection means (8,9), preferably the connection means (8,9) are formed in the internal wall (8) of the proximal nasal portion (21) of the main body (20).

4. Nebulizer according to claim 3, wherein the connection means (8,9) comprise a radial expansion (9) of the internal wall (8) of the proximal nasal portion (21) of the main body (20) and a recess (29) arranged in said radial expansion (9) for receiving the gasket (7) and the mesh member (6).

5. Nebulizer according to claim 3 or 4, wherein the radial expansion (9) of the internal wall (8) comprises at least one first air passage (10) and the wall (8) of the proximal nasal portion (21) comprises at least a second air passage (10') in fluid communication with the ambient atmosphere for equilibrating the gas pressure in the nasal cavity of the user with the atmospheric pressure when the proximal nasal portion (21) is inserted in said nasal cavity.

6. Nebulizer according to any of the preceding claims, wherein the rear surface (6b) of said mesh member (6) is arranged in close proximity to the upper end (2) of the pump shaft (23), preferably the mesh member (6) is maintained in contact with the extremity of said upper end (2) by means of the gasket (7).

7. Nebulizer according to any of the preceding claims, wherein the lower end (3) of the pump (23) is in the deepest part (11) of the liquid suction chamber (12) of the vessel (1).

8. Nebulizer according to any of the preceding claims, wherein at least one hole (24) of the mesh member (6) is configured in such a manner that the liquid passing through said hole (24) produces a conical-shape (40) nebulization, and the shape and size of said proximal nasal portion (21), and the position of the mesh member (6) with respect to the outlet (22) are chosen in order to completely eject the nebulization cone (40) outside said proximal nasal portion (21) through the outlet (22).

9. Nebulizer according to any of the preceding claims, wherein the mesh member (6) comprises several traversing holes (24) extending between the rear and front surfaces (6a, 6b) of said mesh member (6).

10. Nebulizer according to any of the preceding claims, wherein the upper part (13) of said liquid suction chamber (12) is shaped in order to be directly connected to a disposable cartridge (14) for filling liquid into the suction chamber (12) of a vessel (1).

11. Nebulizer according to any of the preceding claims, wherein the proximal nasal portion (21) is bell-shaped.

12. Nebulizer according to any of the preceding claims, wherein the proximal nasal portion (21) comprises an external (15b) and an internal (15a) coaxially-arranged bell-shaped portions spaced one from the other by an collecting chamber (16) for collecting liquids and/or residues, preferably the internal (15a) bell-shaped portion comprises an opening (50) for evacuating liquid and/or residues present in said internal (15a) bell-shaped portion into the collecting chamber (16).

13. Nebulizer according to any of the preceding claims, wherein the main body (20) further comprises an electronic unit (25) for controlling the vibrator (5) and a battery unit (26) for supplying electric power at least to the vibrator (5).

14. Nebulizer according to any of the preceding claims, wherein the mesh member (6) is a plate.

15. Nebulizer according to any of the preceding claims, wherein the main body (20) has a proximal nasal portion (21) having a shape and size adapted for being, at least partially, inserted into a nasal cavity of the user.

16. Nebulizer according to any of the preceding claims, wherein the pump device (23, 2, 3, 4,5) is an ultrasonic pump and/or the vibrator (5) is an ultrasonic vibrator.

17. Nebulizer according to any of the preceding claims, wherein the pump device (23, 2, 3, 4, 5) comprises at least one piezo-electric element arranged on the shaft (23).
